# EUROPÄISCHE PATENTANMELDUNG

(11) **EP 3 175 703 A2**
(43) Veröffentlichungstag der Anmeldung: **07.06.2017**
(21) Anmeldenummer: 16002541.7
(22) Anmeldetag: 25.11.2016
(51) Int. Cl.: A01K 47/06, A61M 11/04, A61M 16/00, A61M 16/12, A61M 16/10, A61M 16/06

(54) **ANSAUGVORRICHTUNG ZUR NUTZUNG VON BIENENSTOCKLUFT**

(30) Priorität: 04.12.2015 DE 202015008332 U
(71) Anmelder: Schmiedgen, Jürgen, 09474 Crottendorf OT Waltersdorf (DE)
(72) Erfinder: Schmiedgen, Jürgen, 09474 Crottendorf OT Waltersdorf (DE)
(74) Vertreter: Kietzmann, Manfred

(57) **Zusammenfassung**

Die Erfindung betrifft eine Ansaugvorrichtung (1) zur Nutzung von Bienenstockluft eines Bienenstocks (10) mit einer Einströmöffnung (3) und einer bienenstockabgewandten Ausströmöffnung (4), die Ansaugvorrichtung (1) auf eine Bienenstocköffnung (11) des Bienenstocks (10) aufsetzbar ist, die Einströmöffnung (3) bienenstockseitig angeordnet ist, ihren Abmaßen nach denen der Bienenstocköffnung (11) im Wesentlichen entspricht, die Ausströmöffnung (4) eine um ein Mehrfaches geringere Querschnittsfläche aufweist, als die der Einströmöffnung (3), an der Ausströmöffnung (4) ein Gebläse (7) angeordnet ist, wobei das Gebläse (7) einen Radialventilator aufweist.

## Beschreibung

Die Erfindung betrifft eine Ansaugvorrichtung zur Nutzung von Bienenstockluft, nämlich zum Inhalieren derselben für den Menschen. Derartige Vorrichtungen sind bereits in den achtziger Jahren des letzten Jahrhunderts entwickelt worden, beispielsweise durch Heinrich Hüttner aus Österreich. Eine solche Vorrichtung ist beispielsweise in der DE 10 2012 100 225 A1 beschrieben. Eine ähnliche Vorrichtung geht ebenfalls aus der DE 20 2009 013 545 U1 hervor.

Bekannte Vorrichtungen bestehen aus einer plattenartigen Ansaugvorrichtung aus Holz, welche in passenden Abmaßen plan auf eine Bienenstocköffnung eines Bienenstocks aufgesetzt ist und mit der Bienenstockluft angesaugt werden kann. Die Ansaugvorrichtung weist eine Auflageplatte zum horizontalen Aufsetzen auf den offenen Bienenstock mit einer Öffnung auf und ist so bienenstockseitig mit einer Einströmöffnung versehen. An diese Einströmöffnung schließt sich Bienenstock abgewandt ein Gebläse an, und zwar in Form eines von einem Gehäuse umgebenen handelsüblichen Axialventilators. Das Gebläse ist auf die Auflageplatte aufgesetzt. Die Einströmöffnung hat im Wesentlichen die Größe eines Ventilators des Gebläses, wie erwähnt in Form eines Axialventilators.

Im Wesentlichen zur Einströmöffnung gegenüberliegend ist an einer Ausströmöffnung der Ansaugvorrichtung eine Durchströmvorrichtung in Form eines Schlauches anschließbar, die in eine Abgabeeinrichtung mündet, welche zur Anlegung an eine Nase oder einen Mund eines Menschen vorgesehen ist, eine so genannte Atemmaske. Über diese atmet der Mensch im Rahmen der Anwendung der Vorrichtung Bienenstockluft ein und wieder aus. An der Einströmöffnung ist ferner ein Filter gelegen, der die Ansaugvorrichtung gegenüber dem Bienenstock abgrenzt, sodass sichergestellt ist, dass keine Bienen durch die Ansaugvorrichtung und so in die Durchströmeinrichtung gelangen können. Des Weiteren befindet sich bereits ein Gitter im Deckel des Bienenstockes. Durch Öffnen einer Abdeckung wird das Gitter freigelegt und die Auflageplatte kann aufgesetzt werden. Ferner ist das Gebläse regelbar.

Es hat sich herausgestellt, dass die Strömungsgeschwindigkeit an der Einströmöffnung, welche im Wesentlichen die Größe des Ventilators besitzt, einen Wert aufweist, der durchschnittliche Strömungsgeschwindigkeiten innerhalb des Bienenstocks bei weitem übersteigt und sich in den Wabengassen sogar noch vergrößern kann. Bei ungünstiger Anordnung von Waben kann sich diese Geschwindigkeit in einer oder mehreren Wabengassen noch wesentlich erhöhen.

Bei dem Einsatz bekannter Axialventilatoren kommt es ferner dazu, dass die angesaugte Luft das elektromagnetische Feld des Motors des Ventilators durchdringt, wenn die Bienenstockluft durch die Einströmöffnung über das Gebläse angesaugt und in die Durchströmeinrichtung geleitet wird. Bedingt der Bauart von Axialventilatoren kommt es des Weiteren zu Verwirbelungen / turbulenten Strömungen, welche die Bienen im Bienenstock negativ beeinflussen können, so beispielsweise den Stressfaktor erhöhen und die Kommunikation der Bienen untereinander negativ beeinflussen.

Ebenfalls hat sich herausgestellt, dass an der Ansaugvorrichtung aufgrund der Temperatur und Feuchtigkeitsunterschiede von Atemluft, Bienenstockluft und Umgebungsluft das Problem auftritt, dass sich innerhalb der vorbeschriebenen Vorrichtung Kondensat bildet. Die Kondensatbildung führt zu erheblichen Problemen in der Anwendungshygiene.

Bei einer bisherigen Holzkonstruktion kann es sogar zu Schimmelbildung kommen. Elektrische und elektronische Bauteile sind durch Kondensatbildung erheblich gefährdet. Es kommt zu Zersetzungen von Lötstellen und Bauteilen. Dadurch kann die gesundheitliche Wirkung der Bienenstocklufttherapie beeinträchtigt werden.

Des Weiteren kommt es in Folge der Kondensatbildung zu Auswaschungen wertvoller Inhaltsstoffe der Bienenstockluft.

Ferner hat sich herausgestellt, dass bei einem Ausatmen der Bienenstockluft durch den Menschen durch den geringen Gegendruck, der durch das Gebläse erzeugt wird, ein Teil der Ausatemluft zurück in den Schlauch geatmet wird. Dies wirkt sich ebenfalls in Bezug auf die Anwendungshygiene und in Bezug auf ein Behandlungsergebnis nachteilig aus, weil die Gefahr besteht, dass der die Vorrichtung anwendende Mensch verbrauchte Luft teilweise wieder einatmet.

Die Aufgabe der Erfindung besteht darin, eine optimierte Vorrichtung zur Nutzung von Bienenstockluft bereitzustellen. Dabei ergeben sich verschiedene Unteraufgaben, so eine verbesserte Anwendungshygiene, bei welcher eine Kondensatbildung reduziert ist und bei der die Bienen behindernde turbulente Strömungen / Verwirbelungen im Bienenstock bei einem Einsatz der Ansaugvorrichtung vermindert werden.

Die Aufgabe bzw. die Unteraufgaben werden durch eine Ansaugvorrichtung zur Nutzung von Bienenstockluft eines Bienenstocks mit den Merkmalen der unabhängigen Ansprüche 1, 10 und 16 gelöst, wobei die Unteransprüche weitere erfindungsgemäße Ausgestaltungsvarianten beinhalten.

Danach weist die erfindungsgemäße Ansaugvorrichtung eine Einströmöffnung und eine Ausströmöffnung auf. Die Ansaugvorrichtung ist dabei auf eine Bienenstocköffnung des Bienenstocks aufsetzbar. Im Wesentlichen erfolgt das Aufsetzen in bekannter Weise horizontal von oben auf den geöffneten Bienenstock. Die Einströmöffnung ist bienenstockseitig angeordnet. Sie entspricht aber im Wesentlichen ihren Abmaßen nach denen der Bienenstocköffnung, was bedeutet, dass die Einströmöffnung alle oder nahezu alle Wabengassen abdeckt. Ferner ist vorgesehen, dass die Ausströmöffnung eine um ein Mehrfaches geringere Querschnittsfläche aufweist, als die der Einströmöffnung. An der bienenstockabgewandten Ausströmöffnung ist ein Gebläse angeordnet. Das Gebläse weist erfindungsgemäß einen Radialventilator auf.

Aufgrund des Einsatzes des Radialventilators kommt es bei einem funktionsgemäßen Einsatz der Ansaugvorrichtung nicht zu relevanten Verwirbelungen. Der Ansaugvorgang ist gleichmäßiger. Die Luft wird in ein Gehäuse des Gebläses geführt, welches den medizinischen Anforderungen genügt, was bei den herkömmlichen Axialventilatoren, die in der Regel Lüfter für Personalcomputer oder ähnliche Vorrichtungen sind, nicht der Fall ist.

Zur Gestaltung der Einströmöffnung ist ferner Folgendes von Bedeutung. Wenn die durch die Einströmöffnung geschaffene Ansaugfläche nicht die vorab dargestellten Abmaße aufweist, kommt es zu einer partiellen Ansaugung und Nachströmung der Luft über ein Flugloch des Bienenstocks, ohne dass die Luft sich genügend mit den Inhaltsstoffen im Bienenstock anreichern kann. Von der erfindungsgemäßen Ansaugvorrichtung werden alle oder nahezu alle Wabengassen erfasst, sodass die vorgenannten Nachteile nicht auftreten. Ferner ist so eine gleichmäßige Ansaugung der Bienenstockluft gewährt.

Die Ausströmöffnung weist, wie eingangs dargestellt, eine um ein Mehrfaches geringere Querschnittsfläche auf, als die der Einströmöffnung. Ihre Querschnittsfläche ist an die Vorgaben des Gebläses angepasst. Der Querschnitt beträgt in der Regel zwischen 20 bis 40 mm.

An der Ausströmöffnung oder an dem Gebläse selbst ist eine Durchströmvorrichtung mit einer Ausgabevorrichtung oder nur eine Ausgabevorrichtung anschließbar, sodass sichergestellt ist, dass ein Mensch die Bienenstockluft leicht inhalieren kann. So bildet die Ansaugvorrichtung mit der anschließbaren Durchströmvorrichtung eine funktionell zusammenwirkende Baugruppe. An der Ansaugvorrichtung können bei Bedarf aber auch beliebige in ihrer Funktion passende Durchströmvorrichtungen mit Ausgabevorrichtungen angeschlossen werden oder nur Ausgabevorrichtungen. Es ist jeweils nur sicherzustellen, dass eine Inhalation durch den Menschen gewährleistet ist, was grundsätzlich auch gewährleistet sein kann, wenn der Mensch im Bereich der Ausströmöffnung die Bienenstockluft einatmet.

Weiterhin kann vorgesehen sein, dass die anordnenbare Durchströmvorrichtung variabel beheizbar ist, so ein beheizbarer variabler Schlauch, und die Durchströmvorrichtung im Betriebszustand eine regelbare Arbeitstemperatur von 35 bis 37 Grad aufweist, sodass es nicht mehr zu Kondensationserscheinungen kommt bzw. dies im Bereich der Durchströmvorrichtung minimiert ist.

Vorteilhafterweise ist die Ansaugvorrichtung eine sich zur Ausströmöffnung verjüngende ein- oder mehrstückige Haube, an deren Basis die Einströmöffnung gelegen ist.

Des Weiteren kann vorgesehen sein, dass die Ansaugvorrichtung aus einem sich zur Ausströmöffnung verjüngenden Ansaugtrichter und einem Gehäuse besteht, welcher das Gebläse mit einer Steuereinheit / Steuerung aufnimmt und lösbar mit dem Ansaugtrichter verbunden ist. Als Steuereinheit / Steuerung ist eine elektronische Steuerung vorgesehen, mit welcher sowohl die Drehzahl des Radialventilators regelbar ist, als auch die Temperatur der anordnenbaren Durchströmvorrichtung. Die Temperatur der anordnenbaren Durchströmvorrichtung und/oder die Drehzahl des Gebläses und damit der Volumenstrom der Bienenstockluft können auch in Abhängigkeit von durch Sensoren ermittelten Eigenschaften der Bienenstockluft, wie den Vorbeschriebenen oder dem Kohlenmonoxidgehalt und/oder Luftfeuchtigkeit, geregelt werden und zwar im Rahmen einer bekannten automatischen Steuerung.

In einer weiteren möglichen Ausgestaltung der Erfindung ist das Gebläse, das im Bereich oder an der Ausströmöffnung angeordnet ist, reversibel befestigt. Die Reversibilität gewährleistet ein einfaches Auswechseln und Reinigen des Gebläses und des Ansaugtrichters sowie der weiteren Bauteile.

Die Ansaugvorrichtung ist stets in passenden Abmaßen auf einen Bienenstock aufsetzbar. Sie weist, wie vorbeschrieben, vorteilhafter Weise die Form einer Haube auf, wobei als Haube vielfältige Formen erfindungsgemäß in Betracht kommen, so Domartige oder Pyramidenförmige. Es ist lediglich eine in Bezug auf die Umgebungsbedingungen, wie Form und Lage des Bienenstocks strömungsgünstige Form zu wählen.

Vorteilhafterweise liegen Mittelpunkte der Querschnittsflächen der Einströmöffnung und der Ausströmöffnung auf einer gedachten Lotrechten, so einer Höhe h, wenn beispielsweise die Ansaugvorrichtung die Form eines Kreiskegelstumpfes oder auch eines Pyramidenstumpfes besitzt.

Die Haubenform ist aber erfindungsgemäß nicht auf die vorgenannten Körper beschränkt. So sind auch andere Körperformen denkbar.

Ebenfalls ist erfindungsgemäß vorgesehen, dass - unabhängig der Abmaße der Einströmöffnung - die erfindungsgemäße Ansaugvorrichtung, so wie sie vorbeschrieben ist, ganz oder teilweise doppelwandig ausgestaltet ist. Dabei ist eine äußere Hülle der Ansaugvorrichtung ganz oder zumindest teilweise doppelwandig.

Die Ansaugvorrichtung zur Nutzung von Bienenstockluft eines Bienenstocks weist dabei, wie bereits dargestellt, eine Einströmöffnung und eine bienenstockabgewandte Ausströmöffnung auf. Die Ansaugvorrichtung ist auf eine Bienenstocköffnung des Bienenstocks aufsetzbar, wobei die Einströmöffnung bienenstockseitig angeordnet ist. An der Ausströmöffnung ist ein Gebläse angeordnet und deren Hülle ist ganz oder teilweise doppelwandig, wobei bei einer teilweisen Doppelwandigkeit entweder nur einzelne Elemente der Ansaugvorrichtung doppelwandig sind oder einzelne Elemente zumindest teilweise doppelwandig gestaltet sind. Wie bekannt, ist an der Ausströmöffnung oder an dem Gebläse eine Durchströmvorrichtung mit einer Ausgabevorrichtung oder nur eine Ausgabevorrichtung, wie bekannt, anschließbar.

Die Doppelwandigkeit gewährleistet eine thermische Isolation zwischen Bienenluft und Temperatur in einem Therapieraum und vermindert zumindest oder verhindert sogar die eingangs erläuterte Kondensatbildung mit den dargestellten nachteiligen Folgen. Ein in diesem Zusammenhang verwendeter Kunststoff ist bereits ein schlechter Wärmeleiter, würde aber nach einer Zeit auch einen Temperaturangleich zulassen, was zu einer Kondensatbildung führen würde.

Weist die Ansaugvorrichtung die Form einer Haube auf, so ist zumindest eine Mantelfläche der Haube doppelwandig ausgestaltet, sodass ein Großteil der Fläche der Ansaugvorrichtung doppelwandig ausgestaltet ist.

Wenn die Ansaugvorrichtung einen Ansaugtrichter aufweist, so ist vorgesehen, dass der Ansaugtrichter doppelwandig ist. Zumindest jedoch sollen Mantelflächen des Ansaugtrichters doppelwandig ausgestaltet sein.

In weiterer Ausgestaltung der Erfindung ist vorgesehen, dass ein durch die Doppelwandigkeit gebildeter Zwischenraum luftreduziert ist oder ein technisches Vakuum aufweist. Dadurch wird die thermisch isolierende Funktion verstärkt.

Ferner hat sich herausgestellt, dass beim Ausatmen durch den geringen Gegendruck, der durch das Gebläse erzeugt wird, ein Teil der Ausatemluft zurück in den Schlauch geatmet wird. Dies kann verhindert werden, indem über ein oder mehrere Sensoren ein durch das Gebläse erzeugter Betriebsdruck in der Durchströmvorrichtung gemessen wird. Bei einer Abweichung eines Istwertes von einem vordefinierten Sollwert kann der im Gebläse angeordnete Ventilator im Ausatmungsvorgang hochgeregelt werden, sodass sich dessen Drehzahl erhöht. Dies kann jedoch Bienen stören, sodass in weiterer bevorzugter Ausgestaltung der Erfindung die Durchströmvorrichtung oder die Ausgabevorrichtung ein Ventil aufweist, welches die Bienenstockluft nur in Richtung der Ausgabevorrichtung bzw. des Menschen strömen lässt und in einem Ausatmungsvorgang des Menschen schließt und eine Membrane sodann nach außen öffnet, sodass die verbrauchte Bienenstockluft an eine Umgebung der Ansaugvorrichtung, so einen den Bienenstock umgebenden Raum, abgegeben wird.

Als Ausgabevorrichtung kommt jedwede für die Luftaufnahme des Menschen geeignete Vorrichtung in Betracht, vorteilhafter Weise ein Mund- und/oder Nasenstück.

Erfindungsgemäß wird so eine optimierte Vorrichtung zur Nutzung von Bienenstockluft bereitgestellt. Bei dieser ist ferner die Anwendungshygiene verbessert, eine Kondensatbildung reduzierbar. Ebenfalls können die Bienen behindernde turbulente Strömungen / Verwirbelungen im Bienenstock bei einem Einsatz der Ansaugvorrichtung vermindert werden.

Die Erfindung wird nachfolgend anhand eines Ausführungsbeispiels unter Bezugnahme auf die Figuren weiter erläutert. Dabei ergeben sich weitere Vorteile, Merkmale und Ausgestaltungen der Erfindung.

Es zeigen:
**Fig. 1** eine schematische Darstellung der Ansaugvorrichtung in Schrägperspektive mit angeschlossener Durchströmvorrichtung mit und Ausgabevorrichtung,
**Fig. 2** eine schematische Darstellung der Haube der Ansaugvorrichtung.

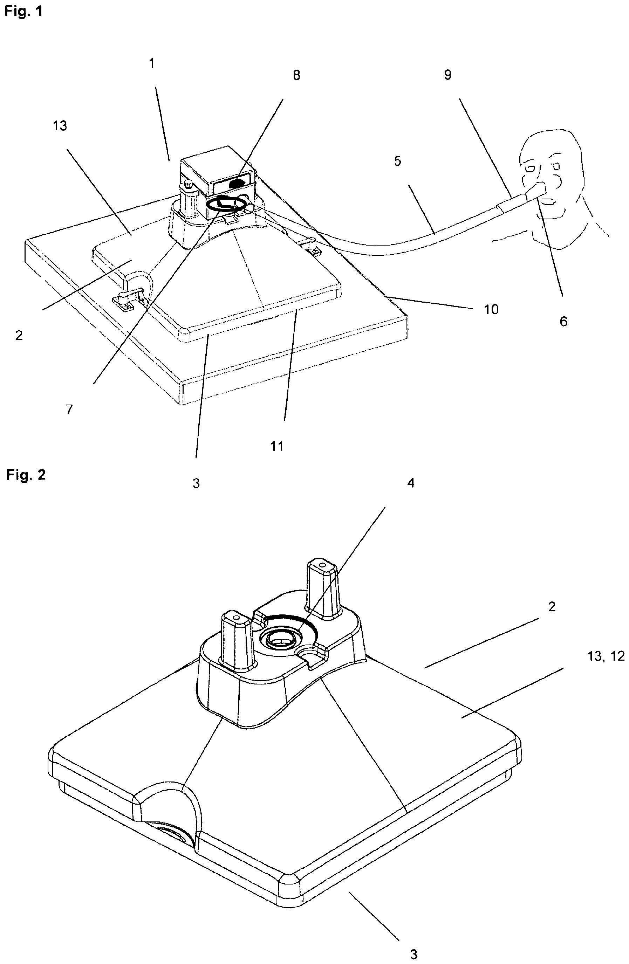

Danach weist die erfindungsgemäße Ansaugvorrichtung 1 eine Einströmöffnung **3** und eine Ausströmöffnung **4** auf. Im Ausführungsbeispiel ist die Ansaugvorrichtung eine sich zur Ausströmöffnung verjüngende trichterförmige Haube **2,** an deren Basis die Einströmöffnung **3** gelegen ist. Die Ansaugvorrichtung **1** besitzt demgemäß einen Ansaugtrichter **12.** Die Mantelfläche **13** des Ansaugtrichters **12** ist doppelwandig. Die Materialstärke von jeweiligen Wänden beträgt ca. 2 mm, der Abstand zwischen einer Innen- und einer Außenwand beträgt ca. 16 mm, **Fig. 1, 2****.**

Die Ansaugvorrichtung **1** ist im Ausführungsbeispiel auf eine Bienenstocköffnung **11** eines Bienenstocks **10** aufgesetzt, **Fig. 1****.** Die bienenstockseitig angeordnete Einströmöffnung **3** entspricht ihren Abmaßen nach im Wesentlichen denen der Bienenstocköffnung **11 -** sie überragt die Bienenstocköffnung geringfügig, um eine Auflage zu gewähren. Die Einströmöffnung **3** deckt so alle Wabengassen des Bienenstocks **10** ab.

An der bienenstockabgewandten Ausströmöffnung **4** ist ein Gebläse **7** in Form eines Radialventilators angeordnet, der in einem Gehäuse auf den Ansaugtrichter **13** aufgesteckt und reversibel arretiert ist. Im Gehäuse ist ferner eine Steuerung **8** angeordnet. Die Steuerung **8** dient zur manuellen Regulierung der Drehzahl des Radialventilators als Gebläse **7.** An dem Gebläse **7** ist eine Durchströmvorrichtung **5** mit einer Ausgabevorrichtung **6** anschließbar und zur besseren Darstellung angeordnet. Bei funktionsgerechter Anordnung der Durchströmvorrichtung **5** an das Gebläse **7** ist über die Steuerung **8** zudem die Temperatur der Durchströmvorrichtung **5** regelbar. Im Ausführungsbeispiel ist die Durchströmvorrichtung **5** ein beheizbarer Schlauch, an dem als Ausgabevorrichtung **6** ein übliches Nasen-/Mundstück für einen die Bienenstockluft inhalierenden Menschen angeschlossen ist.

Des Weiteren weist der Schlauch ein Ventil **9** auf, welches die aus dem Bienenstock **10** mittels des Gebläses **7** angesaugte Bienenstockluft nur in Richtung der Ausgabevorrichtung bzw. des die Bienenstockluft inhalierenden Menschen strömen lässt und bei einem Ausatmungsvorgang des inhalierenden Menschen schließt und sodann eine Membrane nach außen öffnet, sodass die verbrauchte Bienenstockluft an einen den Bienenstock umgebenden Raum abgegeben werden kann.

### Bezugszeichenliste:

- 1.: Ansaugvorrichtung
- 2.: Haube
- 3.: Einströmöffnung
- 4.: Ausströmöffnung
- 5.: Durchströmvorrichtung
- 6.: Ausgabevorrichtung
- 7.: Gebläse
- 8.: Steuerung
- 9.: Ventil
- 10.: Bienenstock
- 11.: Bienenstocköffnung
- 12.: Ansaugtrichter
- 13.: Mantelfläche

## Patentansprüche

1. Ansaugvorrichtung (1) zur Nutzung von Bienenstockluft eines Bienenstocks (10) mit einer Einströmöffnung (3) und einer bienenstockabgewandten Ausströmöffnung (4), die Ansaugvorrichtung (1) auf eine Bienenstocköffnung (11) des Bienenstocks (10) aufsetzbar ist, die Einströmöffnung (3) bienenstockseitig angeordnet ist, ihren Abmaßen nach denen der Bienenstocköffnung (11) im Wesentlichen entspricht, die Ausströmöffnung (4) eine um ein Mehrfaches geringere Querschnittsfläche aufweist, als die der Einströmöffnung (3), an der Ausströmöffnung (4) ein Gebläse (7) angeordnet ist, wobei das Gebläse (7) einen Radialventilator aufweist.

2. Ansaugvorrichtung (1) nach Anspruch 1, wobei an der Ausströmöffnung (4) oder an dem Gebläse (7) eine Durchströmvorrichtung (5) mit einer Ausgabevorrichtung (6) oder eine Ausgabevorrichtung (6) anordnenbar ist, wobei die anordnenbare Durchströmvorrichtung (5) vorzugsweise ein variabler Schlauch ist.

3. Ansaugvorrichtung (1) nach einem der vorherigen Ansprüche, wobei die anordnenbare Durchströmvorrichtung (5) beheizbar ist und im Betriebszustand eine regelbare Arbeitstemperatur von 35 bis 37 Grad aufweist.

4. Ansaugvorrichtung (1) nach einem der vorherigen Ansprüche, wobei die Ausgabevorrichtung (6) ein Mund- und/oder Nasenstück ist.

5. Ansaugvorrichtung (1) nach einem der vorherigen Ansprüche, wobei die Ansaugvorrichtung (1) eine sich zur Ausströmöffnung (4) verjüngende ein- oder mehrstückige Haube (2) ist, an deren Basis die Einströmöffnung (3) gelegen ist.

6. Ansaugvorrichtung (1) nach einem der vorherigen Ansprüche, wobei die Ansaugvorrichtung (1) aus einem sich zur Ausströmöffnung (4) verjüngenden Ansaugtrichter (12) und einem Gehäuse besteht, welcher das Gebläse (7) mit einer Steuereinheit (8) aufnimmt und lösbar mit dem Ansaugtrichter (12) verbunden ist.

7. Ansaugvorrichtung (1) nach einem der vorherigen Ansprüche, wobei die Ausströmöffnung (4) einen Querschnitt von 20 bis 40 mm aufweist.

8. Ansaugvorrichtung (1) nach einem der vorherigen Ansprüche, wobei Mittelpunkte der Querschnittsflächen der Einströmöffnung (3) und der Ausströmöffnung (3) auf einer gedachten Lotrechten gelegen sind.

9. Ansaugvorrichtung (1) zur Nutzung von Bienenstockluft eines Bienenstocks (10) mit einer Einströmöffnung (3) und einer bienenstockabgewandten Ausströmöffnung (4), die Ansaugvorrichtung (1) auf eine Bienenstocköffnung (11) des Bienenstocks (10) aufsetzbar ist, die Einströmöffnung (3) bienenstockseitig angeordnet ist, an der Ausströmöffnung (4) ein Gebläse (7) angeordnet ist, wobei eine äußere Hülle der Ansaugvorrichtung (1) ganz oder teilweise doppelwandig ist.

10. Ansaugvorrichtung (1) nach Anspruch 9, wobei die Ansaugvorrichtung (1) die Form einer Haube (2) besitzt und eine Mantelfläche (13) der Haube (2) doppelwandig ist.

11. Ansaugvorrichtung (1) nach Anspruch 9, wobei ein Ansaugtrichter (12) der Ansaugvorrichtung (1) oder seine Mantelfläche (13) doppelwandig ist.

12. Ansaugvorrichtung (1) nach einem der vorherigen Ansprüche 9 bis 11, wobei ein durch die Doppelwandigkeit gebildeter Zwischenraum luftreduziert ist oder ein technisches Vakuum aufweist.

13. Ansaugvorrichtung (1) nach einem der vorherigen Ansprüche 2 bis 12, wobei das Gebläse (7) eine Steuerung (8) zur Regelung des Radialventilators und/oder der Temperatur der anordnenbaren Durchströmvorrichtung (5) aufweist.

14. Ansaugvorrichtung (1) nach Anspruch 13, wobei, die Steuerung (8) Sensoren zur Messung von Eigenschaften der Bienenstockluft aufweist, wodurch die Temperatur der anordnenbaren Durchströmvorrichtung (5) und/oder die Drehzahl des Gebläses (7) und damit der Volumenstrom der Bienenstockluft in Abhängigkeit von durch die Sensoren ermittelten Werte regelbar ist.

15. Ansaugvorrichtung (1) zur Nutzung von Bienenstockluft eines Bienenstocks (10) mit einer Durchströmvorrichtung (5), wobei die Durchströmtvorrichtung (5) ein Ventil (9) aufweist, durch welches die Bienenstockluft nur in Richtung der Ausgabevorrichtung (6) strömbar ist, das im Ausatmungsvorgang schließbar ist und eine Membrane nach außen öffnenbar ist, sodass die verbrauchte Bienenstockluft an eine Umgebung der Ansaugvorrichtung (1) abgegeben werden kann.
